# EUROPEAN PATENT SPECIFICATION

(11) **EP 3 248 577 B1**
(45) Date of publication and mention of the grant of the patent: **01.09.2021**
(21) Application number: 17172998.1
(22) Date of filing: 26.05.2017
(51) Int. Cl.: A61F 11/08, H04R 25/00, A61B 5/12

(54) **METHOD FOR DETERMINING A MEASURE FOR THE SOUND ATTENUATION PROVIDED BY A HEARING PROTECTOR EAR PLUG AS WELL AS A RELATED COMPUTING DEVICE**
VERFAHREN ZUR BESTIMMUNG EINER MESSUNG ZUR GERÄUSCHDÄMPFUNG DURCH EINEN GEHÖRSCHUTZOHRSTÖPSEL SOWIE ZUGEHÖRIGE BERECHNUNGSVORRICHTUNG
PROCÉDÉ PERMETTANT DE DÉTERMINER UNE MESURE DE L'ATTÉNUATION SONORE FOURNIE PAR UN BOUCHON AURICULAIRE DE DISPOSITIF DE PROTECTION AUDITIVE AINSI QU'UN DISPOSITIF INFORMATIQUE ASSOCIÉ

(30) Priority: 25.05.2016 NL 2016825
(43) Date of publication of application: 29.11.2017
(73) Proprietor: Elacin International B.V., 5107 RG Dongen (NL)
(72) Inventor: VERHAVE, Jan Adrianus, 3723 XE Bilthoven (NL); VAN WIJNGAARDEN, Sander Jeroen, 2497 An den Haag (NL); FLESKENS, Bas, 5107 RG Dongen (NL)
(74) Representative: Algemeen Octrooi- en Merkenbureau B.V.

(56) References cited:
- WO-A1-2011/043819
- WO-A1-2012/043819
- US-A- 3 968 334
- US-A- 4 060 701

## Description

The present invention is related to a method for determining a measure for the sound attenuation provided by a hearing protector ear plug. Presently, several method already exist for determining the above described attenuation. One of the most common methods of determining the attenuation is a MIRE (microphone in real ear) measurement, described below.

First, the hearing protector ear plug is amended in such a way that a microphone, i.e. a sensor for measuring the loudness of an audio signal, is placed on the hearing protector ear plug. The microphone is placed in such a way that it faces the inside of the ear canal of the user once the hearing protector ear plug is placed inside the ear of the user.

Subsequently, a known test sound with a predetermined loudness is provided to the hearing protector ear plug placed inside the ear of the user. The test sound is attenuated by the hearing protector ear plug and its loudness is measured by the microphone. The difference in loudness is then caused by the hearing protector ear plug such that the difference is directly related to the attenuation provided by the hearing protector ear plug.

In the art, the above described method is referred to as a microphone in real ear, MIRE, method.

One of the drawbacks of the known method is that it is very cumbersome. A microphone is to be placed on each hearing protector ear plug which is a difficult task. Further, a part of the hearing protector ear plug is to be removed in order to efficiently place the microphone, thereby resulting in less reliable attenuation results.

Document WO 2011/043819 describes a "Method of deriving individual gain compensation curves for hearing aid fitting".

Document US 3968334 describes an "Audiometric method and apparatus for testing the effectiveness of hearing protective devices".

Document US 4060701 describes a "Method for testing acoustical attenuation of hearing protectors".

It is an object of the present invention to overcome the above described drawbacks.

To this end, the invention in a first aspect, provides for a method for determining a measure for a sound attenuation provided by a hearing protector ear plug when worn by a user, said method comprises the performing of at least two measurements, wherein each of said at least two measurements comprises the steps of:
- providing, by a computing device, a two channel test sound using a left audio channel and a right audio channel to a user via a headset;
- receiving, by said computing device, a balance input signal, wherein said balance input signal relates to a perceived loudness of said left audio channel and a perceived loudness of said right audio channel of said two channel test sound by said user, and controlling, by a computing device, amplitudes of said left audio channel and said right audio channel of said two channel test sound provided to said user via said headset based on said received balance input signal;
- receiving, by said computing device, an approval signal, wherein said approval signal indicates that said user perceives a same level of loudness of said left audio channel and said loudness of said right audio channel;
   wherein a first of said at least two measurements is performed when said user is free from said hearing protector ear plug, and a second of said at least two measurements if performed when said user is wearing said hearing protector ear plug in one of its ears;
   said method further comprises, after performing said at least two measurements, the step of:
   - determining said measure for said attenuation provided by said hearing protector ear plug based on said performed at least two measurements,
   wherein said two channel test sound to said user comprises a binaural audio test sound, being a two channel audio test sound evoking a binaural interaction.

It was the insight of the inventors that it is much easier for a user to determine whether the loudness of the two channel test sound received via its left ear, i.e. left channel, is of the same loudness of two channel test sound received via its right ear, i.e. right channel, in case the two channel test sound provided to said user via said headset comprises a binaural audio test sound.

A binaural audio test sound, in the context of the present invention, means that the audio sound provided to a left ear of the user is slightly different from the audio sound provided to the right ear of the user. The inventors have found that the difference in these sounds can evoke a binaural interaction, such as for example a spatial sensation or a so-called binaural beat, and therewith effectively improve user ability to determine perceived loudness of the sound in both ears.

The measure for an attenuation provided by the hearing protector ear plug may be the amount of decibel, dB, of reduction in amplitude or power caused by the hearing protector ear plug.

In accordance with the present invention, the computing device may be any of a tablet, a smart phone, a laptop, a person computer, or the like.

In an example, the binaural audio test sound is such that a frequency, or frequency band, of said audio of said left audio channel is shifted compared to said frequency, or frequency band, of said audio of said right audio channel.

This means that an auditory illusion is perceived when two different pure-tone sine waves, both with for example a frequency of about 500Hz, with less than a 10-20Hz difference between them, are provided to the user dichotically, that is one through each ear. So, if a 505hz pure sinus tone is presented via a left audio channel to a user's left ear, while e 495Hz pure sinus tone is presented via a right audio channel to a user's right each, the user will perceive the auditory illusion of a third tone, in addition to the two pure tones presented to each ear. The third tone may be referred to as a binaural beat, and, in this example, would have a perceived pitch correlating to a frequency of 10Hz, that being the difference between the 505Hz and 495Hz pure tones presented to each ear. The inventors have found that this effect helps the user to determine whether a perceived loudness at its right ear equals a perceived loudness at its left ear.

In other words, the left ear would hear the 505Hz pure sinus tone, the right would hear the 495Hz sinus tone and the binaural aspect, i.e. the 10Hz tone, is being perceived, by the user, in the middle between the left ear and the right ear.

In order to establish the perceived loudness at both ears, the user may determine the middle point of the perceived two channel tests sound. In case the perceived loudness at both ears is the same, the user will think that the sound is coming from a middle point of its head. In case the right ear perceives a stronger sound compared to the left ear, this will shift to the right side of the head of a user.

In a further example, the binaural audio test sound is such that a phase and/or time of said audio of said left audio channel is shifted compared to said phase of said audio of said right audio channel.

This means that, for example, exactly the same two channel test sound is provided to both ears, but then shifted in phase. That is, a sinus type audio test sound may be provided to the left ear and a cosinus, i.e. shifted 90° in phase, is provided to the right ear of the user. Again, this helps the user in determining whether a loudness of both received audio test parts of the audio test sound are approximately the same.

This may also be explained using small frequency noise bands. A frequency noise band is provided to the user via, for example, the left ear and the same frequency noise band, but then 180° shifted in phase, is provided to the right ear. In phase is then a compact signal perceived inside the head of the user and the antiphase if perceived around the head of the user.

In another example, the frequency of said two channel test sound is between 100hz - 1000hz, preferably between 200Hz - 400Hz. The inventors noted that the attenuation in this frequency range should be focussed on, as in this frequency range it is the most hard to actually provide an attenuation using hearing protector ear plugs.

In a further example, the method comprises the performing of three measurements, a first measurement wherein said user is free from said hearing protector ear plug, a second measurement wherein said user is wearing said hearing protector ear plug in one of its ears and a third measurement wherein said user is wearing another hearing protector ear plug in its other ear.

Minimally, two measurements are required. A first measurement in which no ear plugs are worn by a user and a second measurement in which a single hearing protector ear plug, or both hearing protector ear plugs, are worn by the user. Optionally, in case the second measurement was performed with a single hearing protector ear plug, a third measurement may be performed with another hearing protector ear plug worn in the other ear.

In another example, the computing device comprises receiving means for generating a slider on a touch screen of said computing device, which slider is used for receiving said balance input signal.

The slider may thus be used by the user for inputting its results, i.e. whether the perceived loudness of the sound received at a left ear is the same of the perceived loudness of the sound received at a right ear of the user.

The slider may be a one-dimensional slider wherein the user is able to input its perceived binaural effect, or where the user is able to adjust the perceived binaural effect, by sliding the slider from left to right, or vice versa.

The slider may also be a two-dimensional slider, i.e. a matrix, wherein the user is able to provide its input in such a way that a maximum binaural effect is obtained. That is, the user should slide, or point to, a position in the slider in which a maximum binaural interaction is perceived. One axis may then be directed to the loudness of the two channel sound, and the other axis may be directed to the beat-frequency or phase of the two channel sound.

In a second aspect, the invention provides in a computing device arranged for determining a measure for an attenuation provided by a hearing protector ear pug when worn by a user, said computing device being arranged to perform at least two measurements, wherein said computing device comprises:
- provide equipment arranged for providing a two channel test sound to a user via a headset;
- touch screen arranged for receiving a balance input signal, wherein said balance input signal relates to a perceived loudness of said left audio channel and a perceived loudness of said right audio channel of said two channel test sound by said user, and controlling, by said computing device, amplitudes of said left audio channel and said right audio channel of said two channel test sound provided to said user via said headset based on said received balance input signal;
- touch screen further arranged for receiving an approval signal, wherein said approval signal indicates that said user perceives a same level of loudness of said left audio channel and said loudness of said right audio channel;
   wherein said provide equipment and said touch screen are arranged to perform at least two measurements, wherein a first of said at least two measurements is performed when said user is free from said hearing protector ear plug, and a second of said at least two measurements is performed when said user is wearing said hearing protector ear plug in one of its ears;
   said computing device further comprising:
   - determine equipment arranged for determining said measure for said attenuation provided by said hearing protector ear plug based on said performed at least two measurements, wherein said determine equipment is arranged to perform said step of determining after said provide equipment and said touch screen have performed said at least two measurements
   wherein said two channel test sound to said user comprises a binaural audio test sound.

It is noted that the advantages mentioned with respect to the method are similar, or the same, to the advantages with respect to the computing device.

In an example hereof, the binaural audio test sound is such that a frequency of said audio of said left audio channel is shifted compared to said frequency of said audio of said right audio channel.

In a further example, the said binaural audio test sound is such that a phase of said audio of said left audio channel is shifted compared to said phase of said audio of said right audio channel.

In yet another example, the frequency, or frequency band, of said two channel test sound is between 100hz - 1000Hz, preferably between 200Hz - 400Hz.

In a further example, the said device is further arranged for the performing of three measurements, a first measurement wherein said user is free from said hearing protector ear plug, a second measurement wherein said user is wearing said hearing protector ear plug in one of its ears and a third measurement wherein said user is wearing the other hearing protector ear plug in its other ear.

In yet another example, the computing device comprises receiving means for generating a slider on a touch screen of said computing device, which slider is used for receiving said balance input signal.

The above-mentioned and other features and advantages of the invention will be best understood from the following description referring to the attached drawings. In the drawings, like reference numerals denote identical parts or parts performing an identical or comparable function or operation.

The invention is not limited to the particular examples disclosed below in connection with a particular type of computing device.
Figure 1 is an example of a flow chart illustrating the method steps of the present invention.
Figure 2 is an example of a binaural audio test sound, wherein said binaural audio test sound is such that a frequency of said audio of said left audio channel is shifted compared to said frequency of said audio of said right audio channel.
Figure 3 is an example of a binaural audio test sound, wherein said binaural audio test sound is such that a phase of said audio of said left audio channel is shifted compared to said phase of said audio of said right audio channel.
Figure 4 is an example of a computing device according to the present invention.

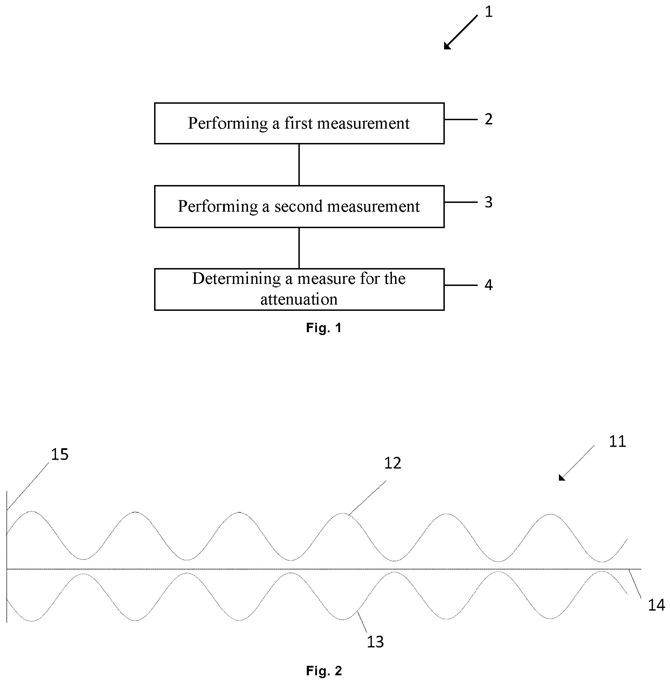

Figure 1 is an example of a flow chart 1 illustrating the method steps of the present invention.

The flow chart 1 thus illustrates a method for determining a measure for a sound attenuation provided by a hearing protector ear plug when worn by a user. As mentioned before, the method comprises the performing of at least two measurements. A reference measurement, being the first measurement 2, and a subsequent second measurement 3. The second measurement 3 does not necessarily be performed immediately after the first measurement 2.The first measurement is a sort of calibration measurement for a particular user. For another user, another calibration measurement is to be performed.

Each of the two measurements 2, 3 comprise several method steps, being. In a first step, a two channel test sound is provided, by a computing device using a left audio channel and a right audio channel to a user via a headset. In a second step, the computing device receives a balance input signal, wherein the balance input signal relates to a perceived loudness of said left audio channel and a perceived loudness of said right audio channel of said two channel test sound by said user, and controlling, by a computing device, amplitudes of said left audio channel and said right audio channel of said two channel test sound provided to said user via said headset based on said received balance input signal. In a third step, the computing device receives an approval signal, wherein said approval signal indicates that said user perceives a same level of loudness of said left audio channel and said loudness of said right audio channel. These three steps may be performed continuously. That is the amplitudes of the of the sound provided through the left and right audio channel may be amended continuously based on input received by the user until the user indicates that a same amount of loudness is perceived in its right and left ear.

Here, the first measurement 2 is performed when the user is free from the hearing protector ear plug. The second measurement 3 is performed when the user is wearing a single hearing protector ear plug in one of its ears.

The method comprises a final step being the determination of the measure for the attenuation 4 based on the two measurements 2, 3.

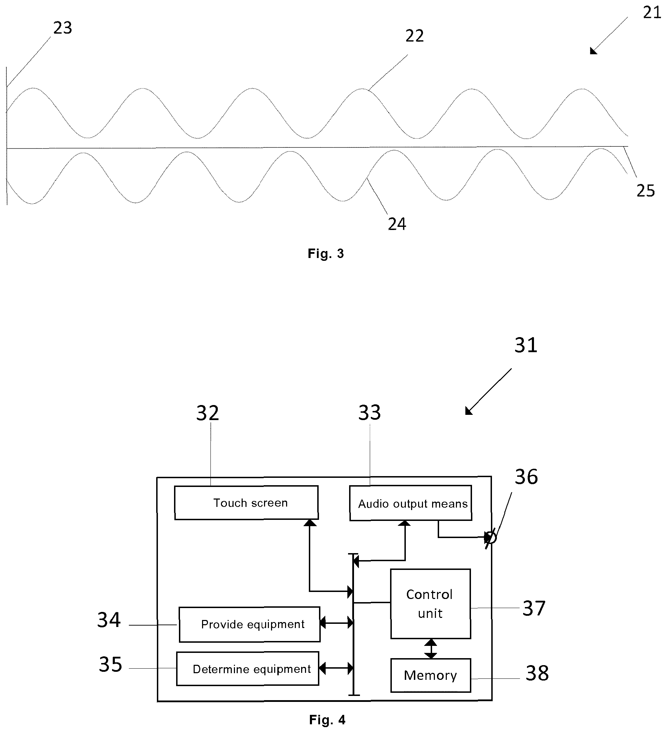

One of the aspects of the present invention is that the two channel test sound that is being provided to the user comprises a binaural audio test sound. This is explained in more detail with respect to figure 2 and figure 3.

Figure 2 is an example of a binaural audio test sound 11, wherein said binaural audio test sound 11 is such that a frequency of said audio of said left audio channel 12 is shifted compared to said frequency of said audio of said right audio channel 13.

Here, the time is indicated with reference numeral 14 and the two channels are indicated with reference numeral 12 and 13. It is shown that the frequencies of both signals are the same, for example about 500 Hz, but the phases of both signals 12, 13 are different. That is, the phase of the audio of the left audio channel 12 is shifted with respect to the phase of the audio of the right audio channel 13. Finally, the amplitude of both signals is indicated with reference numeral 15.

Figure 3 is an example of a binaural audio test sound 21, wherein said binaural audio test sound 21 is such that a phase of said audio of said left audio channel 22 is shifted compared to said phase of said audio of said right audio channel 24.

Here, the time is indicated with reference numeral 25 and the two channels are indicated with reference numeral 22 and 24. It is shown that the frequencies of both signals are shifted slightly, for example shifted about 10 Hz. That is, the frequency of the audio of the left audio channel 22 is shifted with respect to the frequency of the audio of the right audio channel 24. Finally, the amplitude of both signals is indicated with reference numeral 23.

Figure 4 is an example of a computing device 31 according to the present invention.

The computing device 31 comprises a control unit 37, for example a processing unit, connected to a memory 38. The control unit 37 is further connected to a touch screen 32, an audio output means 33, a provide equipment 34 and a determine equipment 35.

The touch screen 32 is arranged to receive a balance input signal from the user, wherein the balance input signal relates to a perceived loudness of the left audio channel and a perceived loudness of the right audio channel of the two channel test sound by the user. The touch screen 32 is further arranged to receive an approval signal, wherein the approval signal indicates that the user perceives a same level of loudness of the left audio channel and the loudness of the right audio channel.

The provide equipment 34 is arranged to provide a two channel test sound using a left audio channel and a right audio channel to a user via a head set. The two channel test sound passes the audio output means 33 and is outputted via the output terminal 36.

The determine equipment is arranged to determine the measure for the attenuation provided by the hearing protector ear plug based on the performed at least two measurements.

Following the above, it is one of the advantage of the invention that a user is better able to determine whether the loudness of a sound perceived at a right ear equals the loudness of a sound perceived at a left ear as the sound comprises a binaural audio test sound.

The binaural audio test sound may be a sound wherein the frequency of the sound of the left audio channel is shifted compared to the frequency of the sound of the right audio channel, or wherein the phase of the sound of the left audio channel is shifted compared to the phase of the sound of the right audio channel.

The present disclosure is not limited to the embodiments as disclosed above, and can be modified and enhanced by those skilled in the art beyond the scope of the present disclosure as disclosed in the appended claims without having to apply inventive skills.

## Claims

1. A method for determining a measure for an attenuation provided by a hearing protector ear plug when worn by a user, said method comprises the performing of at least two measurements (2,3), wherein each of said at least two measurements comprises the steps of:
- providing, by a computing device (31), a two channel test sound using a left audio channel and a right audio channel to a user via a headset;
- receiving, by said computing device (31), a balance input signal, wherein said balance input signal relates to a perceived loudness of said left audio channel and a perceived loudness of said right audio channel of said two channel test sound by said user, and controlling, by said computing device (31), amplitudes of said left audio channel and said right audio channel of said two channel test sound provided to said user via said headset based on said received balance input signal;
- receiving, by said computing device (31), an approval signal, wherein said approval signal indicates that said user perceives a same level of loudness of said left audio channel and said loudness of said right audio channel by an evoked binaural interaction;
wherein a first of said at least two measurements (2) is performed when said user is free from said hearing protector ear plug, and a second of said at least two measurements (3) is performed when said user is wearing said hearing protector ear plug in one of its ears;
wherein said method further comprises, after performing said at least two measurements (2,3), the step of:
- determining said measure for said attenuation (4) provided by said hearing protector ear plug based on said performed at least two measurements (2, 3); and
wherein said two channel test sound to said user comprises a binaural audio test sound, wherein a sound that is provided to a left ear of the user is different from a sound that is provided to the right ear of the user, and wherein said binaural audio test sound is adapted to evoke a binaural interaction.

2. The method according to claim 1, wherein said binaural audio test sound (11) is such that a frequency of said audio of said left audio channel (12) is shifted compared to said frequency of said audio of said right audio channel (13).

3. The method according to any of the previous claims, wherein said binaural audio test sound (21) is such that a phase and/or time of said audio of said left audio channel (22) is shifted compared to said phase of said audio of said right audio channel (24).

4. The method according to any of the previous claims, wherein said frequency of said two channel test sound is between 100Hz - 1000Hz, preferably between 200Hz - 400Hz.

5. The method according to any of the previous claims, wherein said method comprises the performing of three measurements, a first measurement wherein said user is free from said hearing protector ear plug, a second measurement wherein said user is wearing said hearing protector ear plug in one of its ears and a third measurement wherein said user is wearing another hearing protector ear plug in its other ear.

6. The method according to any of the previous claims, wherein said computing device (31) comprises receiving means for generating a slider on a touch screen of said computing device (31), which slider is used for receiving said balance input signal.

7. A computing device (31) arranged for determining a measure for an attenuation (4) provided by a hearing protector ear plug when worn by a user, said computing device (31) being arranged to perform at least two measurements, wherein said computing device (31) comprises:
- a provide equipment (34) arranged for providing a two channel test sound to a user via a headset; and
- a touch screen (32) arranged for receiving a balance input signal,
wherein said balance input signal relates to a perceived loudness of said left audio channel and a perceived loudness of said right audio channel of said two channel test sound by said user, and controlling, by said computing device (31), amplitudes of said left audio channel and said right audio channel of said two channel test sound provided to said user via said headset based on said received balance input signal;
- wherein said touch screen (32) is further arranged for receiving an approval signal, wherein said approval signal indicates that said user perceives a same level of loudness of said left audio channel and said loudness of said right audio channel;
wherein said provide equipment (34) and said touch screen (32) are arranged to perform at least two measurements, wherein a first of said at least two measurements is performed when said user is free from said hearing protector ear plug, and a second of said at least two measurements is performed when said user is wearing said hearing protector ear plug in one of its ears;
wherein said computing device (31) further comprises:
- a determine equipment (35) arranged for determining said measure for said attenuation provided by said hearing protector ear plug based on said performed at least two measurements, wherein said determine equipment (35) is arranged to perform said step of determining after said provide equipment (34) and said touch screen (32) have performed said at least two measurements; and
wherein said two channel test sound to said user comprises a binaural audio test sound, wherein a sound for providing to a left ear of the user is different from a sound for providing to the right ear of the user, and wherein said binaural audio test sound is adapted to evoke a binaural interaction.

8. The computing device according to claim 7, wherein said binaural audio test sound is such that a frequency of said audio of said left audio channel is shifted compared to said frequency of said audio of said right audio channel.

9. The computing device according to any of the claims 7 - 8, wherein said binaural audio test sound is such that a phase of said audio of said left audio channel is shifted compared to said phase of said audio of said right audio channel.

10. The computing device according to any of the claims 7 - 9, wherein said frequency of said two channel test sound is between 100Hz - 1000Hz.

11. The computing device according to any of the claims 7 - 10, wherein said device is further arranged for the performing of three measurements, a first measurement wherein said user is free from said hearing protector ear plug, a second measurement wherein said user is wearing said hearing protector ear plug in one of its ears and a third measurement wherein said user is wearing another hearing protector ear plug in its other ear.

12. The computing device according to any of the claims 7 - 11, wherein said computing device comprises receiving means for generating a slider on the touch screen of said computing device, which slider is used for receiving said balance input signal.

## Patentansprüche

1. Verfahren zum Bestimmen eines Maßes für eine Dämpfung, die durch einen Gehörschutzstöpsel bereitgestellt wird, wenn er von einem Benutzer getragen wird, wobei das Verfahren die Durchführung von wenigstens zwei Messungen (2, 3) umfasst, wobei jede der wenigstens zwei Messungen die Schritte umfasst:
- Bereitstellen, durch eine Computervorrichtung (31), eines zweikanaligen Testtons unter Verwendung eines linken Audiokanals und eines rechten Audiokanals für einen Benutzer über ein Headset;
- Empfangen, durch die Computervorrichtung (31), eines Balance-Eingabesignals, wobei sich das Balance-Eingabesignal auf eine wahrgenommene Lautstärke des linken Audiokanals und eine wahrgenommene Lautstärke des rechten Audiokanals des Zweikanal-Testtons durch den Benutzer bezieht, und Steuern, durch die Computervorrichtung (31), der Amplituden des linken Audiokanals und des rechten Audiokanals des Zweikanal-Testtons, die dem Benutzer über das Headset bereitgestellt werden, basierend auf dem empfangenen Balance-Eingabesignal;
- Empfangen, durch die Computervorrichtung (31), eines Genehmigungssignals, wobei das Genehmigungssignal anzeigt, dass der Benutzer durch eine evozierte binaurale Interaktion einen gleichen Pegel der Lautstärke des linken Audiokanals und der Lautstärke des rechten Audiokanals wahrnimmt;
wobei eine erste der wenigstens zwei Messungen (2) durchgeführt wird, wenn der Benutzer den Gehörschutzstöpsel nicht trägt, und eine zweite der wenigstens zwei Messungen (3) durchgeführt wird, wenn der Benutzer den Gehörschutzstöpsel in einem seiner Ohren trägt;
wobei das Verfahren nach Durchführung der wenigstens zwei Messungen (2, 3) ferner den Schritt umfasst:
- Bestimmen des Maßes der Dämpfung (4), die durch den Gehörschutzstöpsel bereitgestellt wird, basierend auf den durchgeführten wenigstens zwei Messungen (2, 3); und
wobei der Zweikanal-Testton für den Benutzer einen binauralen Audiotestton umfasst, wobei ein Ton, der an ein linkes Ohr des Benutzers bereitgestellt wird, sich von einem Ton unterscheidet, der an das rechte Ohr des Benutzers bereitgestellt wird, und wobei der binaurale Audiotestton dafür ausgelegt ist, eine binaurale Interaktion zu evozieren.

2. Verfahren gemäß Anspruch 1, wobei der binaurale Audiotestton (11) derart ist, dass eine Frequenz des Tons des linken Audiokanals (12) im Vergleich zur Frequenz des Tons des rechten Audiokanals (13) verschoben ist.

3. Verfahren gemäß einem der vorstehenden Ansprüche, wobei der binaurale Audiotestton (21) derart ist, dass eine Phase und/oder Zeit des Tons des linken Audiokanals (22) im Vergleich zur Phase des Tons des rechten Audiokanals (24) verschoben ist.

4. Verfahren gemäß einem der vorstehenden Ansprüche, wobei die Frequenz des Zweikanal-Testtons zwischen 100Hz-1000Hz, vorzugsweise zwischen 200Hz-400Hz liegt.

5. Verfahren gemäß einem der vorstehenden Ansprüche, wobei das Verfahren die Durchführung von drei Messungen umfasst, eine erste Messung, bei der der Benutzer den Gehörschutzstöpsel nicht trägt, eine zweite Messung, bei der der Benutzer den Gehörschutzstöpsel in einem seiner Ohren trägt, und eine dritte Messung, bei der der Benutzer einen weiteren Gehörschutzstöpsel in seinem anderen Ohr trägt.

6. Verfahren gemäß einem der vorstehenden Ansprüche, wobei die Computervorrichtung (31) Empfangsmittel zum Erzeugen eines Schiebers auf einem Berührungsbildschirm der Computervorrichtung (31) umfasst, wobei der Schieber zum Empfangen des Balance-Eingabesignals verwendet wird.

7. Computervorrichtung (31), die dafür ausgelegt ist, ein Maß für eine Dämpfung (4) zu bestimmen, die durch einen Gehörschutzstöpsel bereitgestellt wird, wenn er von einem Benutzer getragen wird, wobei die Computervorrichtung (31) dafür ausgelegt ist, wenigstens zwei Messungen durchzuführen, wobei die Computervorrichtung (31) umfasst:
- eine Bereitstellungsausrüstung (34), die dafür ausgelegt ist, einen Zweikanal-Testton über ein Headset an einen Benutzer bereitzustellen; und
- einen Berührungsbildschirm (32), der dafür ausgelegt ist, ein Balance-Eingabesignal zu empfangen,
wobei sich das Balance-Eingabesignal auf eine wahrgenommene Lautstärke des linken Audiokanals und eine wahrgenommene Lautstärke des rechten Audiokanals des Zweikanal-Testtons durch den Benutzer bezieht, und Steuern, durch die Computervorrichtung (31), der Amplituden des linken Audiokanals und des rechten Audiokanals des Zweikanal-Testtons, die dem Benutzer über das Headset bereitgestellt werden, basierend auf dem empfangenen Balance-Eingabesignal; wobei der Berührungsbildschirm (32) ferner dafür ausgelegt ist, ein Genehmigungssignal zu empfangen, wobei das Genehmigungssignal anzeigt, dass der Benutzer einen gleichen Pegel der Lautstärke des linken Audiokanals und der Lautstärke des rechten Audiokanals wahrnimmt;
wobei die Bereitstellungsausrüstung (34) und der Berührungsbildschirm (32) dafür ausgelegt sind, wenigstens zwei Messungen durchzuführen, wobei eine erste der wenigstens zwei Messungen durchgeführt wird, wenn der Benutzer den Gehörschutzstöpsel nicht trägt, und eine zweite der wenigstens zwei Messungen durchgeführt wird, wenn der Benutzer den Gehörschutzstöpsel in einem seiner Ohren trägt;
wobei die Computervorrichtung (31) ferner umfasst:
- eine Bestimmungsausrüstung (35), die dafür ausgelegt ist, das Maß der Dämpfung, die durch den Gehörschutzstöpsel bereitgestellt wird, basierend auf den durchgeführten wenigstens zwei Messungen zu bestimmen, wobei die Bestimmungsausrüstung (35) dafür ausgelegt ist, den Schritt des Bestimmens durchzuführen, nachdem die Bereitstellungsausrüstung (34) und der Berührungsbildschirm (32) die wenigstens zwei Messungen durchgeführt haben; und wobei der Zweikanal-Testton für den Benutzer einen binauralen Audiotestton umfasst, wobei ein Ton zum Bereitstellen an ein linkes Ohr des Benutzers sich von einem Ton zum Bereitstellen an das rechte Ohr des Benutzers unterscheidet und wobei der binaurale Audiotestton dafür ausgelegt ist, eine binaurale Interaktion zu evozieren.

8. Computervorrichtung gemäß Anspruch 7, wobei der binaurale Audiotestton derart ist, dass eine Frequenz des Tons des linken Audiokanals im Vergleich zur Frequenz des Tons des rechten Audiokanals verschoben ist.

9. Computervorrichtung gemäß einem der Ansprüche 7-8, wobei der binaurale Audiotestton derart ist, dass eine Phase des Tons des linken Audiokanals im Vergleich zur Phase des Tons des rechten Audiokanals verschoben ist.

10. Computervorrichtung gemäß einem der Ansprüche 7-9, wobei die Frequenz des Zweikanal-Testtons zwischen 100Hz-1000Hz liegt.

11. Computervorrichtung gemäß einem der Ansprüche 7-10, wobei die Vorrichtung ferner dafür ausgelegt ist, drei Messungen durchzuführen, eine erste Messung, bei der der Benutzer den Gehörschutzstöpsel nicht trägt, eine zweite Messung, bei der der Benutzer den Gehörschutzstöpsel in einem seiner Ohren trägt, und eine dritte Messung, bei der der Benutzer einen weiteren Gehörschutzstöpsel in seinem anderen Ohr trägt.

12. Computervorrichtung gemäß einem der Ansprüche 7-11, wobei die Computervorrichtung Empfangsmittel zum Erzeugen eines Schiebers auf dem Berührungsbildschirm der Computervorrichtung umfasst, wobei der Schieber zum Empfangen des Balance-Eingabesignals verwendet wird.

## Revendications

1. Procédé pour déterminer une mesure d'une atténuation fournie par un bouchon d'oreille de protection auditive lorsqu'il est porté par un utilisateur, ledit procédé comprend la réalisation d'au moins deux mesures (2, 3), dans lequel chacune desdites au moins deux mesures comprend les étapes de :
- fourniture, par un dispositif informatique (31), d'un son de test à deux canaux en utilisant un canal audio gauche et un canal audio droit à un utilisateur par l'intermédiaire d'un casque :
- réception, par ledit dispositif informatique (31), d'un signal d'entrée d'équilibrage, où ledit signal d'entrée d'équilibrage se rapporte à une intensité de son perçue dudit canal audio gauche et à une intensité de son perçue dudit canal audio droit dudit son de test à deux canaux par ledit utilisateur, et commande, par ledit dispositif informatique (31), d'amplitudes dudit canal audio gauche et dudit canal audio droit dudit son de test à deux canaux fourni audit utilisateur par l'intermédiaire dudit casque sur la base dudit signal d'entrée d'équilibrage reçu ;
- réception, par ledit dispositif informatique (31), d'un signal d'approbation, où ledit signal d'approbation indique que ledit utilisateur perçoit un même niveau d'intensité de son dudit canal audio gauche et de ladite intensité de son dudit canal audio droit par une interaction binaurale évoquée ;
dans lequel une première mesure desdites au moins deux mesures (2) est effectuée lorsque ledit utilisateur ne porte pas ledit bouchon d'oreille de protection auditive, et une deuxième mesure desdites au moins deux mesures (3) est effectuée lorsque ledit utilisateur porte ledit bouchon d'oreille de protection auditive dans l'une de ses oreilles ;
dans lequel
ledit procédé comprend en outre, après avoir effectué lesdites au moins deux mesures (2, 3), l'étape de :
- détermination de ladite mesure de ladite atténuation (4) fournie par ledit bouchon d'oreille de protection auditive sur la base desdites au moins deux mesures effectuées (2,3) ; et
dans lequel ledit son de test à deux canaux pour ledit utilisateur comprend un son de test audio binaural, dans lequel un son qui est fourni à une oreille gauche de l'utilisateur est différent d'un son qui est fourni à l'oreille droite de l'utilisateur, et dans lequel ledit son de test audio binaural est adapté pour évoquer une interaction binaurale.

2. Procédé selon la revendication 1, dans lequel ledit son de test audio binaural (11) est tel qu'une fréquence dudit audio dudit canal audio gauche (12) est décalée par rapport à ladite fréquence dudit audio dudit canal audio droit (13).

3. Procédé selon l'une des revendications précédentes, dans lequel ledit son de test audio binaural (21) est tel qu'une phase et/ou un temps dudit audio dudit canal audio gauche (22) est/sont décalé(e)(s) par rapport à ladite phase dudit audio dudit canal audio droit (24).

4. Procédé selon l'une des revendications précédentes, dans lequel ladite fréquence dudit son de test à deux canaux est comprise entre 100 Hz et 1000 Hz, de préférence entre 200 Hz et 400 Hz.

5. Procédé selon l'une des revendications précédentes, dans lequel ledit procédé comprend la réalisation de trois mesures, une première mesure où ledit utilisateur ne porte pas ledit bouchon d'oreille de protection auditive, une deuxième mesure où ledit utilisateur porte ledit bouchon d'oreille de protection auditive dans l'une de ses oreilles et une troisième mesure où ledit utilisateur porte un autre bouchon d'oreille de protection auditive dans son autre oreille.

6. Procédé selon l'une des revendications précédentes, dans lequel ledit dispositif informatique (31) comprend des moyens de réception pour générer un curseur sur un écran tactile dudit dispositif informatique (31), lequel curseur est utilisé pour recevoir ledit signal d'entrée d'équilibrage.

7. Dispositif informatique (31) agencé pour déterminer une mesure d'une atténuation (4) fournie par un bouchon d'oreille de protection auditive lorsqu'il est porté par un utilisateur, ledit dispositif informatique (31) étant agencé pour effectuer au moins deux mesures, dans lequel ledit dispositif informatique (31) comprend :
- un équipement fournisseur (34) agencé pour fournir un son de test à deux canaux à un utilisateur par l'intermédiaire d'un casque ; et
- un écran tactile (32) agencé pour recevoir un signal d'entrée d'équilibrage, dans lequel ledit signal d'entrée d'équilibrage se rapporte à une intensité de son perçue dudit canal audio gauche et à une intensité de son perçue dudit canal audio droit dudit son de test à deux canaux par ledit utilisateur, et commander, par ledit dispositif informatique (31), des amplitudes dudit canal audio gauche et dudit canal audio droit dudit son de test à deux canaux fourni audit utilisateur par l'intermédiaire dudit casque sur la base dudit signal d'entrée d'équilibrage reçu ;
dans lequel ledit écran tactile (32) est en outre agencé pour recevoir un signal d'approbation, dans lequel ledit signal d'approbation indique que ledit utilisateur perçoit un même niveau d'intensité de son dudit canal audio gauche et de ladite intensité de son dudit canal audio droit ;
dans lequel ledit équipement fournisseur (34) et ledit écran tactile (32) sont agencés pour effectuer au moins deux mesures, dans lequel une première mesure desdites au moins deux mesures est effectuée lorsque ledit utilisateur ne porte pas ledit bouchon d'oreille de protection auditive, et une deuxième mesure desdites au moins deux mesures est effectuée lorsque ledit utilisateur porte ledit bouchon d'oreille de protection auditive dans l'une de ses oreilles ;
dans lequel ledit dispositif informatique (31) comprend en outre :
- un équipement de détermination (35) agencé pour déterminer ladite mesure de ladite atténuation fournie par ledit bouchon d'oreille de protection auditive sur la base desdites au moins deux mesures effectuées, dans lequel ledit équipement de détermination (35) est agencé pour effectuer ladite étape de détermination après que ledit équipement fournisseur (34) et ledit écran tactile (32) ont effectué lesdites au moins deux mesures ; et
dans lequel ledit son de test à deux canaux pour ledit utilisateur comprend un son de test audio binaural, dans lequel un son destiné à une oreille gauche de l'utilisateur est différent d'un son destiné à l'oreille droite de l'utilisateur, et dans lequel ledit son de test audio binaural est adapté pour évoquer une interaction binaurale.

8. Dispositif informatique selon la revendication 7, dans lequel ledit son de test audio binaural est tel qu'une fréquence dudit audio dudit canal audio gauche est décalée par rapport à ladite fréquence dudit audio dudit canal audio droit.

9. Dispositif informatique selon l'une des revendications 7 et 8, dans lequel ledit son de test audio binaural est tel qu'une phase dudit audio dudit canal audio gauche est décalée par rapport à ladite phase dudit audio dudit canal audio droit.

10. Dispositif informatique selon l'une des revendications 7 à 9, dans lequel ladite fréquence dudit son de test à deux canaux est comprise entre 100 Hz et 1000 Hz.

11. Dispositif informatique selon l'une des revendications 7 à 10, dans lequel ledit dispositif est en outre agencé pour la réalisation de trois mesures, une première mesure où ledit utilisateur ne porte pas ledit bouchon d'oreille de protection auditive, une deuxième mesure où ledit utilisateur porte ledit bouchon d'oreille de protection auditive dans l'une de ses oreilles et une troisième mesure où ledit utilisateur porte un autre bouchon d'oreille de protection auditive dans son autre oreille.

12. Dispositif informatique selon l'une des revendications 7 à 11, dans lequel ledit dispositif informatique comprend des moyens de réception pour générer un curseur sur l'écran tactile dudit dispositif informatique, lequel curseur est utilisé pour recevoir ledit signal d'entrée d'équilibrage.
